# EUROPEAN PATENT SPECIFICATION

(11) **EP 2 343 029 B1**
(45) Date of publication and mention of the grant of the patent: **06.05.2015**
(21) Application number: 11150076.5
(22) Date of filing: 04.01.2011
(51) Int. Cl.: A61F 2/16

(54) **Intraocular lens injection instrument**
Einspritzinstrument für eine Intraokularlinse
Instrument d'injection de lentille intraoculaire

(30) Priority: 09.01.2010 JP 2010003468; 01.09.2010 JP 2010196199
(43) Date of publication of application: 13.07.2011
(73) Proprietor: Nidek Co., Ltd., Gamagori-shi Aichi 443-0038 (JP)
(72) Inventor: Nagasaka, Shinji, Gamagori-shi Aichi 443-0038 (JP); Sunada, Tsutomu, Gamagori-shi Aichi 443-0038 (JP); Natsume, Akiyoshi, Gamagori-shi Aichi 443-0038 (JP)
(74) Representative: Prüfer & Partner GbR European Patent Attorneys

(56) References cited:
- EP-A1- 2 055 265
- EP-A1- 2 074 963
- WO-A1-96/28122
- WO-A1-2011/061791
- WO-A2-2005/023154

## Description

### Technical Field

The present invention relates to an intraocular lens injection instrument for injecting an intraocular lens (IOL) into an eye.

### Background Art

As one of operative treatment instruments for cataract, heretofore, there has generally been used an intraocular lens injection instrument (hereinafter, referred to as an injector) for injecting an intraocular lens (IOL) into an eye from which a clouded lens has been removed. Such injector has a tapered front end. Accordingly, when the IOL is placed in the injector and then pushed toward the front end of the injector by use of a push rod called a plunger, the IOL is folded into a smaller size along the shape of an inner wall of the injector. Since the IOL in a small folded state is to be injected in an eye, an incision to be formed in the eye can be made as small in diameter as possible. This reduces a burden on a patient and prevents the generation of aftereffect such as postoperative astigmatism (JP 8(1996)-24282 A). In injecting the IOL into the eye by use of the above injector, an optical part and support parts of the folded IOL have to be completely injected into the eye through the front end of the injector.

However, when the optical part is folded in the injector, a rear one of the support parts may be twisted and hence held as it is in the injector. In this case, when the optical part is gradually opened while being injected into the eye, the optical part may exhibit unintended behaviors due to such twisting of the rear support part. To avoid this defect, the twisted rear support part has to be quickly injected out of the injector. However, when the optical part which will be opened first in the eye is further pushed by the plunger, attention should be given to avoid detachment of a tip end of the plunger from the optical part. It is therefore difficult to quickly push out the entire IOL.

Further, in order to insert an injection part of the injector into the eye through the incision formed in a cornea of the eye, a cutout called a bevel is formed at the front end of the injector. In the case where such injector is used, the injector's front end is inserted in the eye with the bevel (the slat surface) facing down and the folded IOL is injected into the eye. However, when the IOL is folded by the injector, a folding direction is roughly determined but the IOL is not always folded in the same way. Accordingly, an opening direction of the IOL after injected out of the injector's front end is hard to be determined. In some cases, therefore, an operator has to adjust an opening state of the IOL by rotating the injector about its axis while observing the behavior of the IOL being gradually opened.

EP 2074 963 A1 describes an intraocular lens insertion tool with a tool body for accommodating an intraocular lens and adapted to insert into an eye the intraocular lens through displacement of the lens in an axial forward direction by a plunging member, and to push out the lens through an insertion tube section disposed at an axial distal end of the tool body. Therein, the plunger is inserted into the lens insertion tool prior to placement of the lens, such that the support part of the lens can be placed in a respective notch of the plunger.
WO 2005/023154 A2 describes a preloaded IOL-injector, which includes a retainer for releasably holding an IOL in an unstressed state. Again, in the proximity of the plunger tip, a recessed area is provided rearwardly of the tip, which is generally aligned with a trailing haptic of the IOL held by the retainer.
EP 2 055 265 A1 describes an intraocular lens injection instrument comprising a lens holding part for holding an intraocular lens with a push-out unit axially movable in the cylindrical main unit to push the IOL out of the lens holding part. The push-out unit comprises a push rod, a head portion at a distal end of the push rod and a narrow portion rearwardly provided from the distal end of the push rod in the proximity of the tip end. In use, the narrow portion accommodates the support part of the IOL.
WO 96/28122 describes a surgical implantation device for insertion of a deformable intraocular lens into an eye comprising a lens injection device having a plunger with a plunger tip configured to provide a side clearance between the plunger tip and the lens insertion passageway through the surgical implantation device to accommodate the trailing haptic of the deformable intraocular lens to prevent damage thereto during insertion.

### Summary of Invention

The present invention has a purpose to provide an intraocular lens injection instrument capable of stably and quickly injecting and opening an entire IOL including support parts in an eye and stabilizing an opening behavior of the IOL in the eye and also easily injecting the IOL without adjusting an opening state.

### Solution to Problem

This purpose is achieved with a device according to claim 1. One aspect of the invention provides an intraocular lens injection instrument for injecting an intraocular lens (IOL) including an optical part and support parts into an eye by folding the IOL, comprising: a cylinder body part provided, at its front end, with an injection part configured to internally hold the IOL and inject the IOL into the eye through an incision formed in the eye; and a plunger for pushing the IOL out of the injection part, the plunger being placed in the cylinder body part to be movable in an axial direction of the cylinder body part, characterized by a tip end portion of the plunger being formed with a holding part for holding the support parts of the IOL.

Further developments of the present invention are given in the dependent claims.

### Brief Description of Drawings

FIG. 1A is an explanatory view showing a configuration of a one-piece intraocular lens;
FIG. 1B is an explanatory view showing a configuration of a three-piece IOL;
FIGs. 2A and 2B are explanatory views showing an external configuration of a cylinder part of an injector;
FIG. 3A is an explanatory view showing a configuration of a plunger;
FIG. 3B is an enlarged view of a part X in FIG. 3A, but seen from obliquely below in FIG. 3A;
FIG. 4 is a schematic cross sectional view showing an internal structure of the injector;
FIG. 5A to 5C are explanatory views showing an opening motion of the one-piece IOL;
FIGs. 6A and 6B are modified examples of a plunger;
FIG. 7A is an explanatory view showing a configuration of a plunger of an injector in a second embodiment;
FIG. 7B is an enlarged view of a part Y in FIG. 7A;
FIG. 7C is a schematic diagram of an injection part when viewed from a front end thereof; and
FIGs. 8A to 8D are explanatory views showing an opening motion of the three-piece IOL.

### Description of Embodiments

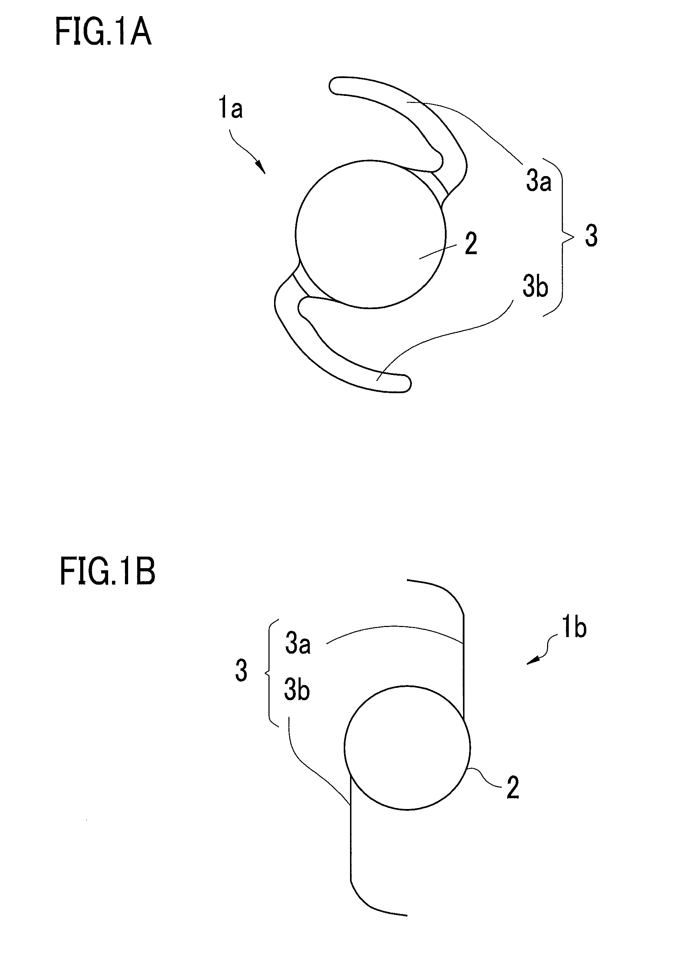

A detailed description of a preferred embodiment of the present invention will now be given referring to the accompanying drawings. FIG. 1A is an explanatory view showing a configuration of a one-piece IOL 1a. FIG. 1B is an explanatory view showing a configuration of a three-piece IOL 1b.

The IOL 1a includes an optical part 2 having a predetermined refractive power and a pair of support parts 3 (a front support part 3a and a rear support part 3b arranged in a pushing direction) for supporting the optical part 2 in an eye. The IOL 1a is constituted of the optical part 2 and the support parts 3 integrally made of a material conventionally used for a foldable soft IOL such as a monomeric material such as HEMA (hydroxyethyl methacrylate) and a composite material of acrylic ester and methacrylate ester by molding or other techniques.

The three-piece IOL 1b shown in FIG. 1B is produced in such a way that an optical part 2 and loop-shaped (C-shaped, J-shaped) support parts 3 each having a free end (a front support part 3a and a rear support part 3b arranged in a pushing direction) are separately produced and then combined in an integral form. The optical part 2 of the three-piece IOL 1b is made of the same material as the one-piece IOL 1a. On the other hand, the support parts 3 are made of a material conventionally used for IOL's support parts of an IOL such as PMMA (polymethacrylate).

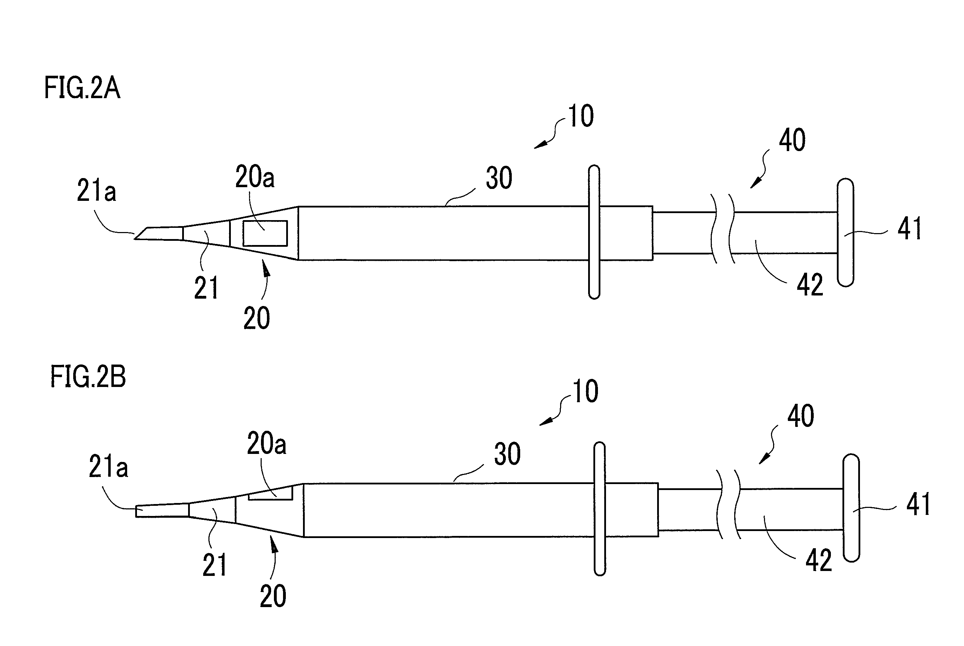

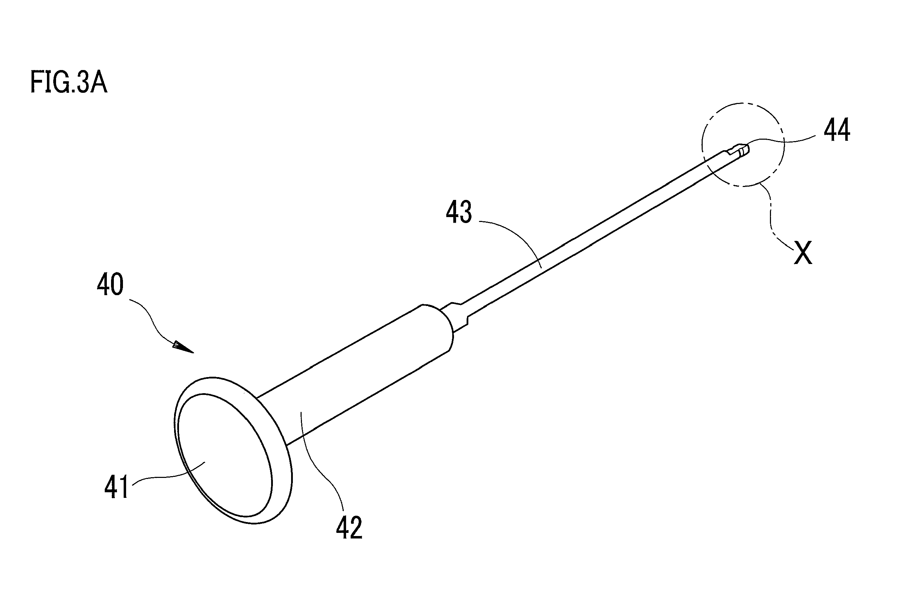

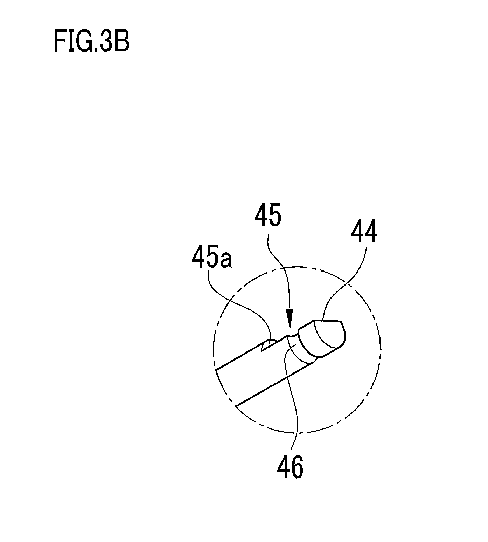

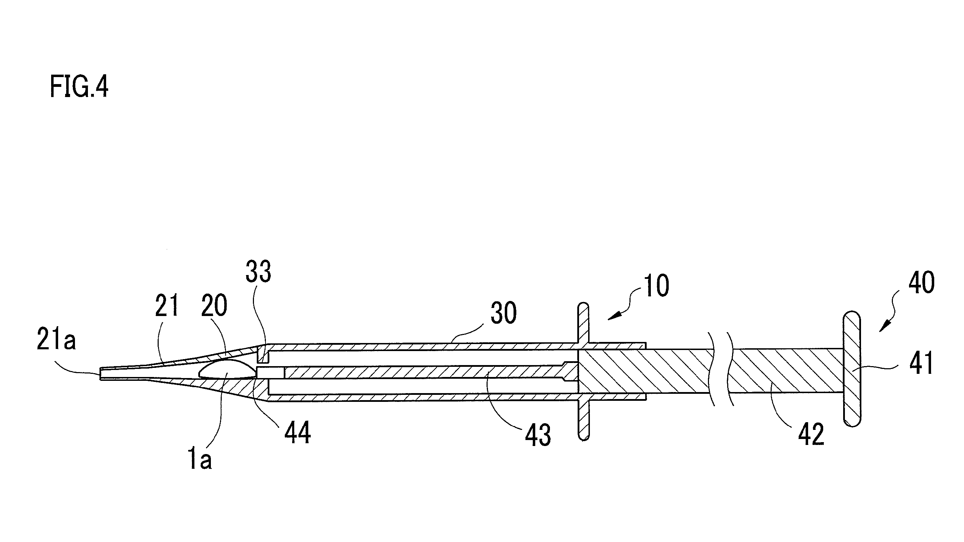

An explanation is given to the configuration of an intraocular lens injection instrument (an injector) 10. The injector 10 described herein is adapted for the one-piece IOL 1a. FIGs. 2A and 2B are schematic diagrams showing an outer appearance of the injector 10; FIG. 2A is a plan view of the injector 10 viewed from above and FIG. 2B is a side view of the same viewed from side. FIG. 3A is an explanatory view showing a configuration of a plunger 40. FIG. 3B is an enlarged view of a part X in FIG. 3A, but seen from obliquely below in FIG. 3A. FIG. 4 is a schematic cross sectional view showing an internal configuration of the injector 10.

The injector 10 includes a injection part 20 in which the IOL 1a is to be placed, a cylinder body part (hereinafter, a "cylinder part") 30 provided with the injection part 20 at a front end, and the plunger 40 movable back and forth with respect to the cylinder part 30 for pushing the IOL 1a.

The injection part 20 includes a tapered injection tube 21 and a lid 20a. The tube 21 has a region whose inner diameter is gradually smaller (thinner) toward a front end 21 a. The IOL 1a is set in the injection part 20 through an opening which appears when the lid 20a is opened. Further, the IOL 1a is folded into a smaller size by passing the interior of the injection tube 21 and then injected out through the front end 21a. The front end 21a of the injection tube 21 is formed with a bevel which is a cutout having a predetermined slant angle with respect to a pushing axis so that the front end 21a is easily injected into a patient's eye through the incision formed in the eye. A slant direction (a cutout direction) of the bevel of the front end 21a is determined according to an opening (spreading or unfolding) direction of the IOL 1a. A relationship between the opening direction of the IOL 1a and the slant direction of the bevel will be explained in detail later.

The hollow cylinder part 30 is internally formed with a centering part 33 for centering the plunger 40. This prevents axis deflection or displacement of the plunger 40 during forward/backward movement and allows a tip end 44 of the plunger 40 to contact with the optical part 2 in a constant state. In the cylinder part 30, the rod-like plunger 40 is inserted to be axially movable through a passage extending through the cylinder part 30 to the front end 21a of the injection part 20. The plunger 40 includes the tip end 44 which comes into contact with the optical part 2 to push the IOL 1a, a press part 41 to be pressed by an operator, an axial base part 42 joined with the press part 41, and a push rod 43 joining the tip end 44 and the axial base part 42. The tip end 44 of the plunger 40 is formed to have such a size (diameter) as to pass through the internal passage of the injection part 20 to push the IOL 1a out of the injection part 20 and as to allow the tip end 44 and the rear support part 3b to pass together through the injection part 20.

The plunger 40 is formed, at the tip end 44 side, with a holding part 45 for holding a part of the rear support part 3b while the tip end 44 is in contact with the optical part 2. The holding part 45 is formed in such a shape as to be made by cutting out a part of the push rod 43 in a direction from the tip end toward a base end. The holding part 45 provided in this manner serves to determine the opening direction of the IOL 1a to one direction about the support part 3b located in the holding part 45.

The holding part 45 is formed on a side in which a proximal end of the rear support part 3b of the IOL 1a set in the injection part 20 is located. The holding part 45 is further formed to provide an opening through which the rear support part 3b can get into between the inner wall of the injection tube 21 and a tip end portion of the push rod 43 while the tip end 44 of the plunger 40 is placed in the injection tube 21.

A plane 45a is formed in a position on axis and apart from the tip end 44 by a predetermined distance toward the base end of the plunger 40 in the present embodiment, i.e., in a position of the holding part 45 side. The plane 45a is formed in a position off a range in which at least a root portion of the rear support part 3b is less warped or deformed (i.e., is rigid) while the tip end 44 is in contact with the optical part 2. This configuration allows the support part 3b pushed by the plane 45a to be pushed out through the front end 21a even when a part of the support part 3b is caught in the holding part 45. It is to be noted that pushing the rear support part 3b by the plane 45a allows more stable injection and opening of the entire IOL into the eye.

In the present embodiment, a groove 46 is formed in a portion of the tip end 44 left as a result of formation of the holding part 45. The groove 46 is used to further hold a part of a distal end portion of the rear support part 3b when the support part 3b is held in the holding part 45 (when the support part 3b is received between the inner wall of the injection tube 21 and the side wall of the tip end portion of the push rod 43). The groove 46 is formed closer to the tip end 44 than the plane 45a. Thus, a part of the distal end portion of the support part 3 is received in the groove 46 in association with a pushing operation of the plunger 40.

With the above configuration, at the time when the optical part 2 pushed out of the injection tube 21 starts to open, the rear support part 3 is held in the injection part 21 at a position of the holding part 45. Thus, the optical part 2 is opened about the support part 3b. Accordingly, irrespective of the folded state of the optical part 2 in the injection tube 21, the opening direction of the optical part 2 is uniformly determined. Further, since a part of the support part 3 is caught by the holding part 45 (or the groove 46), the optical part 2 can be prevented from bursting to separate from the plunger 40 and open even when the optical part 2 opens at high speed. Thus, the opening motion of the IOL 1a is made more stable.

On the other hand, the bevel of the front end 21 a is formed in the slant direction according to the opening direction of the IOL 1a. To be specific, the slant direction of the bevel is determined so that one surface of the optical part 2 faces the bevel (a slant surface of the cutout) when the IOL 1a (the optical part 2) whose opening direction is uniformly determined as mentioned above is completely open outside the injection tube 21. Accordingly, when the optical part 2 is pushed out of the front end 21 with the bevel facing down, the opened optical part 2 comes to a horizontal state with respect to the pushing axis. This configuration allows an operator to appropriately open the IOL 1a easily in the capsule by just pushing the plunger 40 (without rotating the injection part 20).

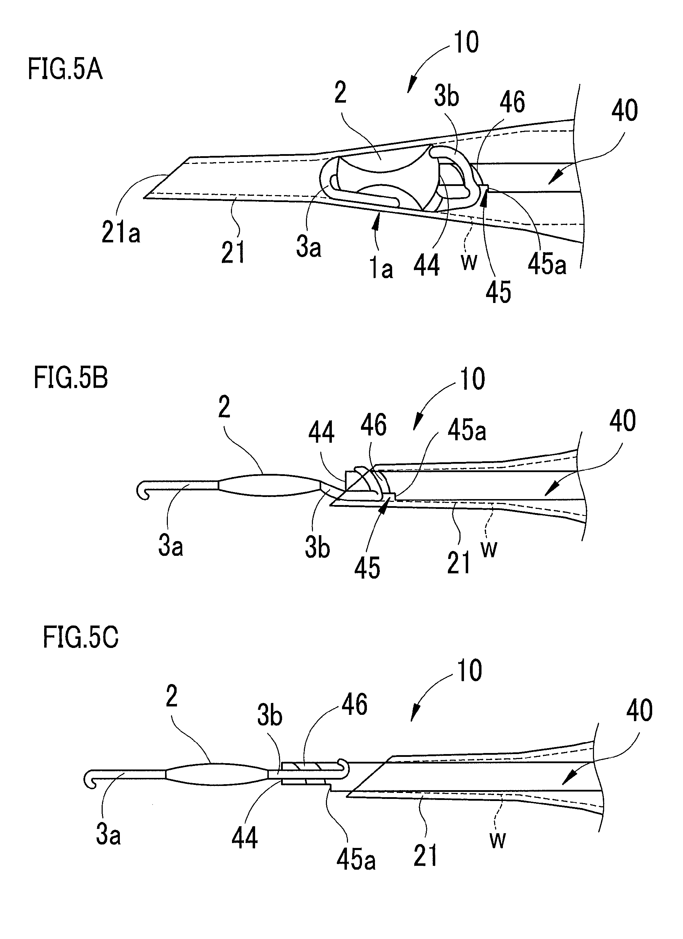

A push-out operation of the IOL 1a using the injector 10 having the aforementioned configuration will be explained below. FIGs. 5A to 5C are explanatory views showing the opening operation of the IOL 1a. Firstly, an operator (a user) puts the IOL 1a with forceps or the like in a predetermined position in the injection part 20 through the opening located under the lid 20a. He/she then inserts the front end 21 a in the eye so that the bevel faces downward along the incision. In this state, when the press part 41 is pushed ahead, a folding position of the rear support part 3b gets into the holding part 45 and a part of the support part 3b is caught in the groove 46 as shown in FIG. 5A. Further, the tip end 44 of the plunger 40 comes into contact with the optical part 2. When the IOL 1a is pushed ahead from this state in the injection part 20, the optical part 2 is gradually folded (rounded) along the inner wall w because the opening diameter of the injection part 20 is narrow. A part of the support part 3b getting into the holding part 45 is gradually caught between the push rod 43 and the inner wall w.

When the optical part 2 is further pushed ahead, the optical part 2 is injected out of the front end 21a and starts to gradually open. At that time, as shown in FIG. 5B, the support part 3b is left in the injection part 20 as remaining caught in the holding part 45 and the groove 46. The optical part 2 is allowed to open in one direction about the support part 3b caught in the holding part 45 and the groove 46. Further, the support part 3b being caught in the holding part 45 can prevent the IOL 1a from bursting to separate from the front end 21 a. On the other hand, when the plane 45a comes into contact with a part of the support part 3b in association with pushing of the IOL 1a, the support part 3b is then pushed by the plane 45a. In this case, the entire IOL 1a is appropriately injected into the eye. Since the bevel of the front end 21a is formed in the slant direction according to the optical part 2 whose opening direction is determined, the IOL 1a is allowed to open stably (without tilting or the like) in the position of (or in front of) an opening of the bevel.

When the plunger 40 is further pushed ahead until the groove 46 and the holding part 45 (the plane 45a) come inside the capsule, the support part 3b separates from the groove 46 and the holding part 45 (the plane 45a) by a restoring force of the support part 3b as shown in FIG. 5C. Thus, the optical part 2 is placed along the capsule by stress applied from the interior of the eye and is appropriately placed in the eye by the support parts 3 (3a and 3b).

As above, the IOL 1a is allowed to open about the support part 3b left in the injection part, so that the opening direction of the IOL 1a is determined in one direction. The slant direction of the bevel of the front end of the injection part 20 is determined in accordance with the opening direction of the IOL 1a. This enables the operator to easily place the IOL 1a in the capsule appropriately by just one push-out operation. When a part of the support part 3b comes into contact with the plane 45a, the entire IOL 1a is appropriately pushed out by the push-out operation of the plunger 40.

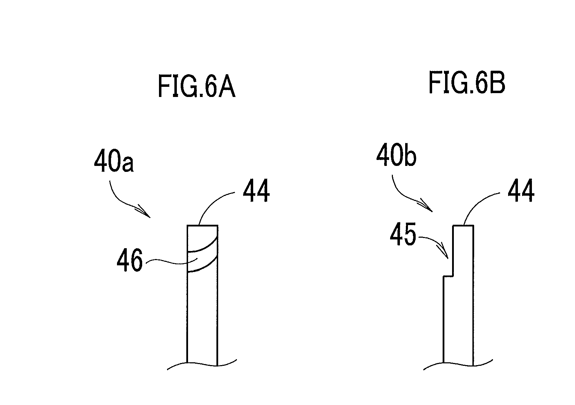

The present embodiment shows the aforementioned example in which the plunger 40 is formed with both the holding part 45 and the groove 46 but is not limited thereto. FIG. 6A is a modified example 1 showing a structure of a plunger 40a. In the case where the opening speed of the IOL is relatively slow, the plunger 40a may be formed with only a groove 46 on a side of a tip end 44. This groove 46 is formed in a position closer to the tip end 44 of the plunger 40a than a position of the support part 3b (a position farthest from the optical part 2) while the tip end 44 is in contact with the optical part 2. This makes it possible to make the opening direction of the IOL stable in one direction by use of the plunger 40a having a simpler configuration.

FIG. 6B is a modified example 2 showing a structure of a plunger 40b. In this example, the plunger 40b is formed with only a holding part 45. In this case, when the optical part 2 opens in the capsule, the opening direction of the IOL 1a is made stable in one direction because a part of the support part 3b is caught in the holding part 45. In another configuration, the injector has only to include a configuration that determines the opening direction of the optical part 2 to one direction about a part of the rear support part 3b when the optical part 2 is to open in the capsule.

In the aforementioned configurations, a pair of support parts 3 of the IOL 1a are arranged one behind the other along the pushing axis. The present invention may be applied to an injector 10 configured that a pair of support parts 3 of an IOL 1a are arranged in a lateral direction with respect to the pushing axis, so that the IOL can be appropriately injected into the eye.

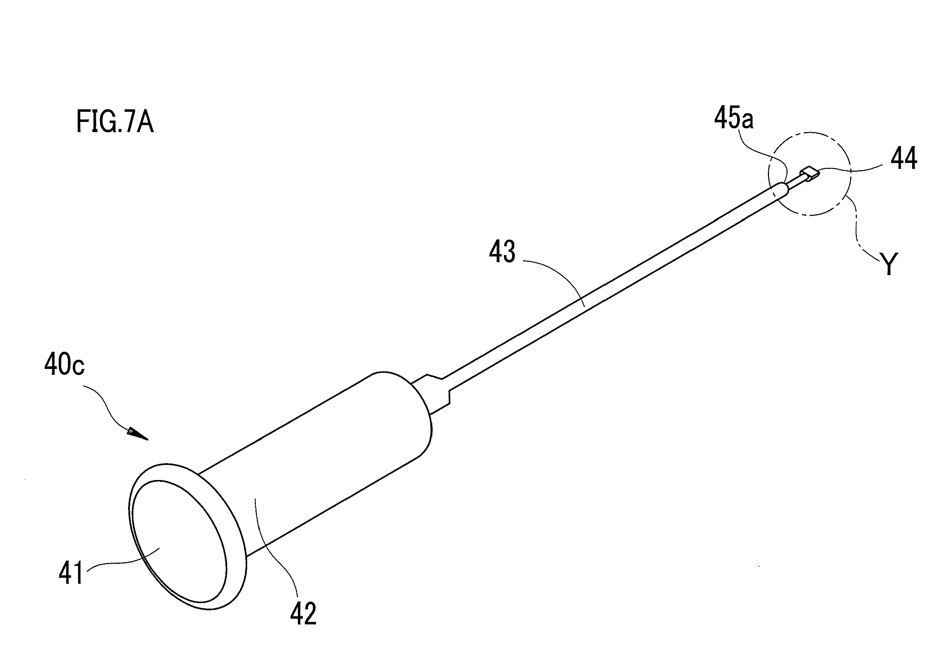

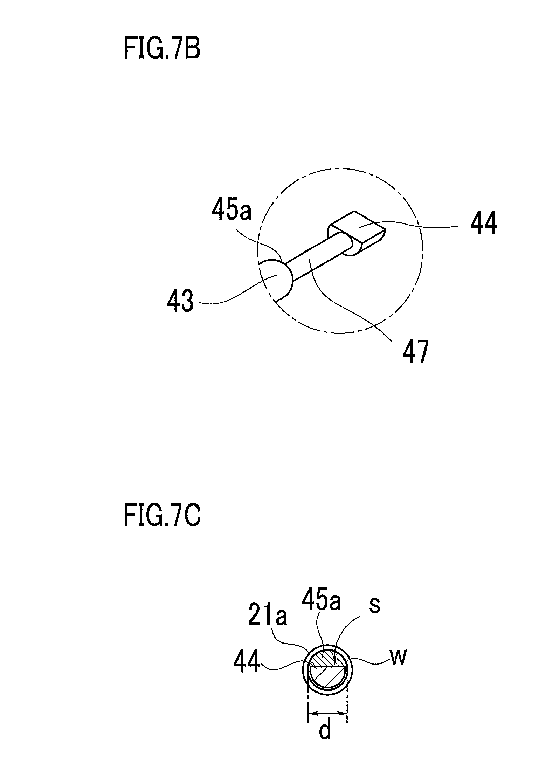

A second embodiment of an injector 10 will be explained below. The injector 10 in this embodiment is mainly adapted for a three-piece IOL 1b but may be used for the one-piece IOL 1a. In the following explanation, identical or similar components to those of the aforementioned injector 10 are given the same reference signs and the details thereof are not explained. FIG. 7A is an explanatory view showing a configuration of a plunger 40c of the injector 10 in the second embodiment. FIG. 7B is an enlarged view of a part Y enclosed with a dotted line in FIG. 7A. FIG. 7C is a schematic view of an injection part 21a viewed from front.

In this embodiment, a plane 45a with which a distal end of the support part 3b will contact is formed in a position on axis (about the axis) and apart from a tip end 44 of the plunger 40c by a predetermined distance toward a basal end while a rear support part 3b is placed in the plunger 40c and the tip end 44 of the plunger 40c is in contact with the optical part 2. When the plane 45a comes into contact with the distal end of the support part 3b, the support part 3b is pushed ahead by the plane 45a by a pushing operation of the plunger 40c and becomes held in the injection tube 21. In this embodiment, as shown in FIG. 7B, a shaft 47 joining the tip end 44 of the plunger 40c with the push rod 43 have a thinner diameter than a diameter (a lateral width) of the tip end 44 and the push rod 43 has almost the same diameter as the opening of the front end 21a. Accordingly, the plane 45a is formed on the axis at a boundary between the push rod 43 and the shaft 47.

To be concrete, the plane 45a is formed between a position on axis corresponding to a maximum diameter of the IOL 1b including the rear support part 3b not being exposed to stress that causes unchanging deformation while the tip end 44 is in contact with the optical part 2 and a position on axis corresponding to the distal end of the rear support part 3b spread out by a pushing operation. It is to be noted that the "unchanging deformation" represents a state that a part of the support part 3b is folded in a reverse direction and damaged or broken and thus the IOL 1b is unable to return to its original shape. Since the position of the plane 45a is determined as above, it is possible to prevent the generation of defects such as breakage of the support part 3b. Further, the plane 45a is formed as a flat surface almost perpendicular to the pushing axis so as to push the rear support part 3b at the same time when pushing the IOL 1b.

As shown in FIG. 7C, the diameter d of the plane 45a is set to be almost equal to the opening of the front end 21a but not limited thereto. The diameter d of the plane 45a has only to be determined to allow the plane 45a to pass through an internal space area of the injection tube 21 and also include a portion with which the distal end of the support part 3b comes into contact. Further, the plane 45a in this embodiment is formed in the entire circumference (360 degrees) of the pushing axis. The plane 45a has only to be formed in a position where the distal end of the support part 3b comes into contact with the plane 45a during at least pushing of the IOL 1b.

On the other hand, the cross section of the tip end 44 has only to have a size (a shape) allowing insertion of both the tip end 44 and the support part 3b through the internal space area of the injection tube 21. In this embodiment, the cross section of the tip end 44 is designed to be almost semicircular. As alternatives, the cross section of the tip end 44 may be determined to provide a space s serving as an escape route to avoid the support part 3b from getting caught between the tip end 44 and the inner wall w of the injection tube 21. In the case of the three-piece IOL, therefore, it is possible to prevent such defects as to cause the support part 3b to be caught and hence broken between the tip end 44 and the injection tube 21.

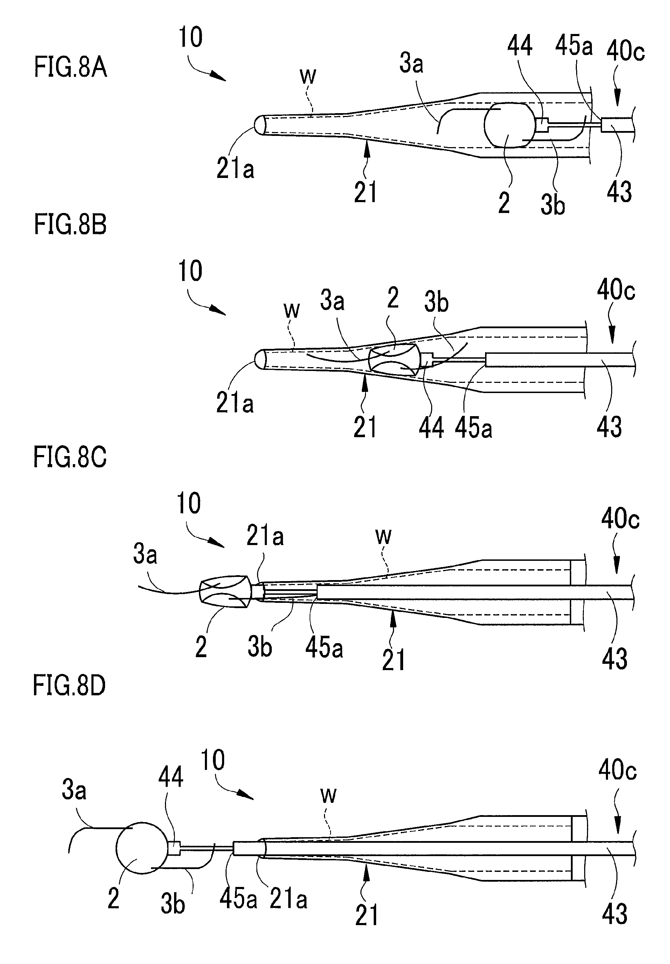

An explanation is further given to an operation for pushing the three-piece IOL 1b by use of the injector 10 including the plunger 40c configured as above. FIGs. 8A to 8D are explanatory views of the pushing operation of the IOL 1b. When the press part 41 is pressed while the IOL 1b is placed in the injection part 20, the tip end 44 comes into contact with the optical part 2 as shown in FIG. 8A. At that time, the rear support part 3b is positioned on the axis of the push-out rod 43. When the press part 41 is further pressed, the IOL 1b enters in the injection part 21 as shown in FIG. 8B. Since the opening diameter of the injection part 21 is narrow, the optical part 2 is folded along the inner wall w.

On the other hand, the distal end of the rear support part 3b touches the inner wall w in association with folding of the optical part 2. From this state, when the press part 41 is further pressed, the support part 3b is gradually spread from a contact point with the inner wall w, serving as a base point, and twisted from the contact point with the inner wall w by a folding motion of the optical part 2. Further, the rear support part 3b is spread out by the pushing operation and thus the distal end of the support part 3b comes into contact with the plane 45a of the holding part. Since the internal space area of the injection part 21 is narrow, the support part 3b spread once in the injection part 21 will not return to its original shape, so that the support part 3b remaining in a twisted state is moved toward the front end 21a.

When the press part 41 is further pressed from a position where the distal end of the support part 3b is in contact with the plane 45a, the optical part 2 pushed out of the front end 21a starts to gradually open in the capsule as shown in FIG. 8C. At that time, the support part 3b is held (enclosed, i.e. held against movement) in the space s while the distal end of the support part 3b remains contact with the plane 45a. Thus, the optical part 2 is allowed to open in one direction about the support part 3b left in the injection part 21. As mentioned above, the slant direction of the bevel of the front end 21a is determined in accordance with the opening direction of the IOL 1b. Thus, the optical part 2 is allowed to stably open in a position of (or in front of) the opening of the bevel.

Even after the optical part 2 is released from the injection part 21, the distal end of the support part 3b remains contact with the plane 45a. When the press part 41 is further pushed ahead after release of the optical part 2, accordingly, the support part 3b is pressed by the plane 45a and quickly injected into the eye as shown in FIG. 8D irrespective of whether the tip end 44 is in contact with the optical part 2. As a result, before stress resulting from twisting of the support part 3b is exerted on the optical part 2, the entire IOL 1b is pushed out of the injection part 20. This makes it possible to prevent unintended behaviors of the optical part 2 previously placed in the eye.

When the plane 45a is pushed out of the front end 21a as mentioned above, the support part 3b separates from the plane 45a by a restoring force and then is placed along the capsule by stress applied by the interior of the eye. The optical part 2 is thus appropriately held in the eye by the support parts (3a and 3b).

Since the rear support part 3b is pushed by the plane 45a of the plunger 40c, the entire IOL 1b is appropriately injected into the capsule before the stress resulting from the twisting of the support part 3b is exerted on the optical part 2. Furthermore, the IOL 1b is stably pushed out by both the tip end 44 and the plane 45a. It is therefore easy for an operator to push out the IOL 1b, thereby positioning the IOL 1b quickly (by one operation) in the eye.

While the presently preferred embodiment of the present invention has been shown and described, it is to be understood that this disclosure is for the purpose of illustration and that various changes and modifications may be made without departing from the scope of the invention as set forth in the appended claims.

## Claims

1. An intraocular lens injection instrument (10) for injecting an intraocular lens (IOL) (1a, 1b) including an optical part (2) and support parts (3) into an eye by folding the IOL, comprising:
a cylinder body part (30) provided, at its front end, with an injection part (20) configured to internally hold the IOL and inject the IOL into the eye through an incision formed in the eye; and
a plunger (40) for pushing the IOL out of the injection part, the plunger including a push rod (43) formed with a tip end (44) and being placed in the cylinder body part to be movable in an axial direction of the cylinder body part,
**characterized by** ' a tip end portion of the push rod including a holding part (45) for catching and holding the support parts of the IOL between a side wall of the tip end portion and an inner wall of the injection part,
the holding part being formed to provide an opening made by cutting out a part of the push rod of the plunger in a direction along an axis from a tip end toward a base end of the plunger to allow a rear support part (3b) of the IOL to get into between the inner wall of the injection part and the side wall of the tip end portion of the push rod for holding a part of the rear support part to uniformly determine an opening direction of the folded optical part (2) irrespective of the folded state of the optical part (2) in the injection part (20), and
the holding part including a plane (45a) formed in a position on axis and apart from the tip end toward the base end of the plunger by a predetermined distance to push out the rear support part (3b) through the front end (21a) of the injection part (20); and wherein
the intraocular lens injection instrument includes a bevel formed in the front end (21a) of the injection part (20), wherein the bevel has a predetermined slant angle with respect to the plunger axis, wherein the bevel is formed in the slant direction according to the opening direction of the IOL, and wherein the bevel is determined by its slant direction to face one surface of the optical part (2) while the IOL is opened outside an injection tube.

2. The IOL injection instrument according to claim 1, wherein the holding part includes a groove (46) formed in the tip end portion of the plunger, for catching a part of the rear support part (3b).

## Patentansprüche

1. Einspritzinstrument (10) für eine Intraokularlinse zum Einspritzen einer Intraokularlinse (IOL) (1a, 1b), die ein optisches Teil (2) sowie Stützteile (3) aufweist, in ein Auge mittels Falten der IOL, mit folgenden Merkmalen:
- ein zylindrischer Hauptteil (30), der an seinem vorderen Ende mit einem Einspritzteil (20) versehen ist, der dafür ausgelegt ist, die IOL in seinem Inneren zu halten und die IOL durch einen in dem Auge gebildeten Schnitt in das Auge einzuspritzen; und
- ein Kolben (40), um die IOL aus dem Einspritzteil hinauszuschieben, wobei der Kolben eine Schubstange (43) aufweist, die mit einem Spitzenende (44) ausgebildet und in dem zylindrischen Hauptteil angeordnet ist, um in einer axialen Richtung des zylindrischen Hauptteils beweglich zu sein, **dadurch gekennzeichnet, dass**
- ein Spitzenendteil der Schubstange ein Halteteil (45) aufweist, um die Stützteile der IOL zu ergreifen und zwischen einer Seitenwand des Spitzenendteils und einer inneren Wand des Einspritzteils zu halten,
- wobei das Halteteil derart ausgebildet ist, dass es eine Öffnung aufweist, die durch ein Ausschneiden eines Teils der Schubstange des Kolbens in einer Richtung entlang einer Achse von einem Spitzenende zu einem Basisende des Kolbens hin hergestellt wird, um es einem hinteren Stützteil (3b) der IOL zu ermöglichen, zwischen die innere Wand des Einspritzteils und die Seitenwand des Spitzenendteils der Schubstange einzutreten, um einen Teil des hinteren Stützteils zu halten, um eine Öffnungsrichtung des gefalteten optischen Teils (2) unabhängig von dem gefalteten Zustand des optischen Teils (2) in dem Einspritzteil (20) einheitlich zu bestimmen, und
- wobei das Halteteil eine Ebene (45a) aufweist, die in einer Position auf Achse und um einen vorgegebenen Abstand von dem Spitzenende weg zu dem Basisende des Kolbens hin ausgebildet ist, um das hintere Stützteil (3b) durch das vordere Ende (21a) des Einspritzteils (20) auszuschieben;
und
- wobei das Einspritzinstrument für eine Intraokularlinse eine in dem vorderen Ende (21a) des Einspritzteils (20) ausgebildete Abschrägung aufweist, wobei die Abschrägung einen vorgegebenen geneigten Winkel in Bezug auf die Kolbenachse aufweist, wobei die Abschrägung in der Neigungsrichtung entsprechend der Öffnungsrichtung der IOL ausgebildet ist, und wobei die Abschrägung durch ihre Neigungsrichtung dafür bestimmt ist, einer Oberfläche des optischen Teils (2) gegenüberzuliegen, während die IOL außerhalb eines Einspritzrohrs geöffnet wird.

2. Einspritzinstrument für eine IOL nach Anspruch 1, wobei das Halteteil eine in dem Spitzenendteil des Kolbens ausgebildete Nut (46) aufweist, um einen Teil des hinteren Stützteils (3b) zu ergreifen.

## Revendications

1. Instrument d'injection d'une lentille intraoculaire (10) permettant d'injecter une lentille intraoculaire (IOL) (1a, 1b) comprenant une partie optique (2) et des parties supports (3) dans un oeil en repliant cette lentille intraoculaire comprenant :
une partie de corps cylindrique (30) équipée à son extrémité frontale d'une partie d'injection (20) conformée pour maintenir la lentille intraoculaire à sa partie interne et l'injecter dans l'oeil par une incision réalisée dans celui-ci, et
un piston (40) permettant de pousser la lentille intraoculaire à l'extérieur de la partie d'injection, ce piston comprenant une tige poussoir (43) ayant une extrémité de tête (44) et positionnée dans la partie de corps cylindrique de façon à pouvoir se déplacer dans la direction axiale de cette partie de corps,
**caractérisé par**
une partie d'extrémité de tête de la tige poussoir comprenant une partie de maintien (45) pour saisir et maintenir les parties supports de la lentille intraoculaire entre la paroi latérale de la partie d'extrémité de tête et la paroi interne de la partie d'injection,
la partie de maintien étant réalisée pour permettre d'obtenir une ouverture effectuée en découpant une partie de la tige poussoir du piston dans une direction s'étendant le long d'un axe partant de l'extrémité de tête vers l'extrémité de base du piston pour permettre à une partie support arrière (3b) de la lentille intraoculaire de s'insérer entre la paroi interne de la partie d'injection et la paroi latérale de la partie d'extrémité de tête de la tige poussoir pour maintenir une partie de la partie support arrière de façon à déterminer uniformément la direction d'ouverture de la partie optique (2) repliée quelque soit l'état replié de la partie optique (2) dans la partie d'injection (20), et
la partie de maintien comprenant un plan (45a) situé dans une position sur l'axe et à une distance prédéfinie de l'extrémité de tête vers l'extrémité de base du piston pour pousser la partie support arrière (3b) au travers de l'extrémité frontale (21a) de la partie d'injection (20), et
l'instrument d'injection d'une lentille intraoculaire comprenant un biseau situé au niveau de l'extrémité frontale (21a) de la partie d'injection (20), ce biseau étant incliné angulairement par rapport à l'axe du piston, ayant une direction d'inclinaison conforme à la direction d'ouverture de la lentille intraoculaire et la direction d'inclinaison du biseau étant déterminée pour être situé en regard d'une surface de la partie optique (2) lorsque la lentille intraoculaire est ouverte à l'extérieur d'un tube d'injection.

2. Instrument d'injection d'une lentille intraoculaire conforme à la revendication 1, dans lequel la partie de maintien comprend une rainure (46) formée dans la partie d'extrémité de tête du piston pour saisir une partie de la partie support arrière (3b).
